# FASCICULE DE BREVET EUROPEEN

(11) **EP 0 579 616 B1**
(45) Date de publication et mention de la délivrance du brevet: **23.10.1996**
(21) Numéro de dépôt: 92905929.3
(22) Date de dépôt: 11.02.1992
(51) Int. Cl.: A61K 35/64

(54) **PROCEDE D'EXTRACTION D'UNE SUBSTANCE BIOLOGIQUE A PARTIR DE LARVES DE BOMBYX MORI ET SUBSTANCE OBTENUE**
METHODE ZUR EXTRAKTION EINER BIOLOGISCHEN SUBSTANZ AUS LEPIDOPTERA-LARVEN UND EINE SOLCH ERHALTENE SUBSTANZ
METHOD FOR EXTRACTING A BIOLOGICAL SUBSTANCE FROM LEPIDOPTERA LARVAS AND SUBSTANCE THUS OBTAINED

(30) Priorité: 11.02.1991 FR 9101705
(43) Date de publication de la demande: 26.01.1994
(73) Titulaire: FERENT, Maurice, F-63960 Veyre-Monton (FR)
(72) Inventeur: FERENT, Maurice, F-63960 Veyre-Monton (FR)
(74) Mandataire: Chanet, Jacques
(86) Numéro de dépôt international: FR9200115
(87) Numéro de publication internationale: WO9213543

(56) Documents cités:
- FR-A- 1 503 586
- FR-A- 2 517 964
- L'INFORMATORE DEL GIOVANE ENTOMOLOGO, vol. 66, 1973, pages 1-4; GASTALDI, F.: 'preparazione a secco di insetti e loro larve mediante liofilizzazione'
- CHEMICAL ABSTRACTS, vol. 102, no. 3, 21 Janvier 1985, Columbus, Ohio, US; abstract no. 20725; PARK, J.R.: 'factors affecting the stractability of protein of silkworm larvae', page 356, colonne 2
- CHEMICAL ABSTRACTS, vol. 111, no. 11, 11 Septembre 1989, Columbus, Ohio, US; abstract no. 93504; ZHANG, G.: 'method for the extraction of odorless protein from silkworm with organic solvents', page 392, colonne 1
- CHEMICAL ABSTRACTS, vol. 73, no. 17, 26 Octobre 1970, Columbus, Ohio, US; abstract no. 85052; TASHIRO, Y.: 'posterior silk gland of bombyx mori (silkworm). IV. Ultracentrifugal analyses of native silk proteins, especially fibroin extracted from the middle silk gland of the mature silkworm', page 119, colonne 1

## Description

La présente invention est du domaine des biotechnologies et a pour objet un procédé d'obtention d'un produit biologique à partir de la larve du ver à soie : BOMBYX MORI, le produit obtenu suivant le procédé et des applications de ce produit aux domaines de la dermo-cosmétique, pharmacie, diététique.

Actuellement, il existe sur le marché de nombreuses matières premières visant ces domaines, d'origine végétale ou animale en particulier mammifères (organiques, placentaire, embryonnaire, sanguine...), ainsi que des extraits de chrysalides, notamment de BOMBYX MORI. Par ailleurs, la larve de BOMBYX MORI est connue par ses qualités nutritionnelles en particulier dans les pays tropicaux et équatoriaux où elle permet de limiter les carences multinutritionnelles à ceux qui en consomment. En effet, sur un plan analytique, cet aliment contient des vitamines, des oligo-éléments, des acides aminés essentiels, des acides gras essentiels en proportions tout a fait intéressantes.

De nombreuses publications ont décrit les techniques de traitement des larves d'ansectes et notamment du BOMBYX MORI.

Pour cerner de manière plus précise l'arrière plan de l'invention, se reporter aux deux publications suivantes.

En premier lieu, un Brevet FR1503586 (BORDAS) décrit la manière d'obtenir des produits cosmétiques à partir de corps adipeux de lépidoptères, le BOMBYX MORI étant cité ; il y a lieu d'observer toutefois que cette publication préconise le traitement de l'"insecte" dans le cas présent au stage de sa métamorphose Nymphe/Chrysalide, soit le 6ème âge, car selon l'auteur l'activité est plus importante que celle qui pourrait être observée sur la larve au 5ème âge, c'est-à-dire à son stade chenille ou larve, avant le coconnage.

La simple observation des analyses citées dans les tableaux permet de constater qu'il n'en est rien et c'est pourquoi il est proposé de traiter l'insecte en entier dans sa phase chenille et uniquement dans la période du 5ème âge. Le traitement peut se faire sur le ver frais ou congelé ou sur le ver lyophilisé par des extractions de type chimique ou/et mécanique pour obtenir les meilleures propriétés pharmacodynamiques (tableaux 1, 2 et 3).

En second lieu, KODONDI, LECLERCQ, BOURGEAY-CAUSSE et la (1987) ont publié dans la revue "Aliments" sous le titre "Intérêt nutritionnel de chenilles et attacidés du Zaire : composition et valeur nutritionnelle", un article fournissant des analyses de larves et leurs modes opératoires ; cet article ne précise pas toutefois à quel stade de développement doivent être prises ces chenilles qui, au demeurant, ne sont pas de larves de BOMBYX MORI.

La démarche inventive du demandeur a consisté, dans un premier temps, à mettre en doute le fait que seul le corps adipeux de la chenille ou de la chrysalide pouvait expliquer les vertus cosmétiques et, dans un deuxième temps, à procéder à de nombreuses analyses précises : il apparaît que, dans le 5ème âge (avant la métamorphose), la chenille possède une quantité très équilibrée d'éléments protéiques, lipidiques, vitaminiques. Dans un troisième temps, les domaines d'application sont élargis à la dermatologie et à la nutritrion.

L'invention, tant dans les conditions du procédé que dans les résultats quantitatifs obtenus est strictement limitée à la larve de BOMBYX MORI élevée dans une magnanerie et nourrie avec des feuilles de mûriers.

Le procédé de l'invention consiste tout d'abord à sélectionner et à récolter les chenilles atteignant le stade dit "de la montée", dans le 5ème âge, c'est à dire à l'age consécutif à la 5ème mue, en général, vers le 8ème jour, parfois plus tard.

Suivant une première variante du procédé, ces chenilles sont, de préférence, et dans cet ordre : broyées, de préférence cryobroyées, séchées, de préférence lyophilisées, et la poudre obtenue tamisée. A l'un quelconque des stades de traitement, le produit peut être congelé, de préférence sous azote liquide en attendant l'étape suivante. On obtient une poudre plus ou moins fine selon le tamis utilisé, plus ou moins homogène selon le broyage, d'odeur végétale, de couleur plus ou moins verte selon la façon dont le produit a été conservé.

Une seconde étape du procédé de l'invention consiste en l'ensemble des étapes consécutives suivantes : centrifugation, broyage, séchage, tamisage.

Une troisième étape consiste en une extraction lipidique par des méthodes chimiques comme la méthode de FOLCHE, ou le CO2 super critique plus ou moins associé à une pompe à co-solvant, ou par l'hexane, ou autre solvant organique.

Une quatrième étape peut consister à séparer par tamisage la glande séricigène et son contenu broyé du reste de la poudre.

Une cinquième étape consiste à effectuer une extraction aqueuse selon des procédés classiques pour obtenir ainsi un résidu délipidé sans soie insoluble dans l'eau. Soumise à hydrolise par des moyens physiques ou chimiques, la substance obtenue représente un hydrolisat d'acides aminés utilisable en cosmétologie et produits d'hygiène corporelle.

Une sixième étape consiste à congeler, en notant que la congélation est possible après l'une quelconque des étapes précitées en attendant l'étape suivante.

Une septième étape consiste en l'adjonction d'additifs tels que : antioxydants, conservateurs, antiseptiques, antibiotiques, huiles essentielles, parfums... pourrant être incorporés à un stade quelconque des extractions dans une substance biologique quelconque issue des extractions afin de la stabiliser, de parfumer...

Une huitième étape consiste à stériliser le produit d'extraction par filtration, par rayonnements ionisants, par gaz ou par procédés chimiques ; on notera que ce traitement est applicable à la susbtance issue de l'une quelconque des étapes précitées.

A titre de variante de la première étape on peut laisser le ver à soie se vider du contenu de leurs tubes digestifs et recueillir, trier et sécher cette substance excrémentielle qui contient des vitamines en grande quantité et en particulier la vitamine B12 ; la substance obtenue est utilisable en nutrition animale et humaine.

A titre de variante du procédé sus-décrit la deuxième étape peut être précédée d'une centrifugation.

A titre de variante on sépare par différence de densité les deux composants de la soie de BOMBYX MORI fibroine et séricine, la soie obtenue pouvant être considérée comme de la soie native possédant un haut pouvoir couvrant quand elle est pulvérulente après tamisage fin, et pouvant être utilisée à ce titre comme poudre de maquillage, ou encore comme charge dans les produits cosmétiques en particulier de maquillage, à titre de fixation de pigment, ou encore être utilisée comme vecteur de principe actif ; un tamisage moins fin, ou plus grossier, permet d'obtenir une poudre légèrement abrasive pouvant être utilisée comme produit de gommage ou de peeling dans le domaine de la dermocosmétologie.

A titre illustratif d'extraction convenant à la substance de l'invention, le Demandeur préconise le mode opératoire suivant:
- mise en suspension de la poudre de ver à soie dans l'eau (soit poudre brute, soit poudre délipidée, soit poudre désoyée, soit poudre délipidée et désoyée),
- macération sous agitation mécanique ou magnétique, par exemple à 30°C pendant 1 heure,
- puis filtration sur verre fritté ou sur papier, sous vide ou sous pression atmosphérique,
- la solution obtenue, dont l'extrait sec est fonction des quantités relatives d'eau et de poudre, peut être stabilisée par adjonction de propylèneglycol (ou autre conservateur), la stabilisation pouvant aussi être réalisée par déssication.

La méthode sus-indiquée s'applique aussi bien à la poudre qu'au broyat frais du ver.

Dans la substance obtenue suivant la variante de la deuxième étape dans laquelle on a conservé au ver à soie ses glandes séricigènes, le rapport protéines (en grammes) sur lipides (en grammes) est supérieur à 4,5, et la somme des quantités de vitamines B5 et PP est supérieure ou égale à 10 mg.

La substance constituant le reliquat du traitement de la quatrième étape à savoir le ver amputé de sa glande séricigène, traité par dissolution dans l'eau, peut être utilisée en cosmétologie et dermatologie et elle possède un pouvoir oxygraphique important à extrèmement important selon la concentration en principes actifs, elle a un effet cicatrisant et un effet de lissage cutané donc antiride par modification du nombre et de la profondeur de rides.

La substance résultant de l'amputation des glandes séricigènes a un rapport protéines (en grammes) sur lipides (en grammes) supérieur à 3,5.

La substance peut être utilisée en dermatologie et en cosmétologie, diluée dans un corps gras ou pur et incorporé dans un excipient plus ou moins gras ou dans un gel ; elle a un effet de restructuration cutanée après agression par exemple le lauryl sulfate ; en outre elle augmente la résistance de la peau à la déformation (test de fermométrie) et a un effet hydratant par reconstitution de la barrière cutanée.

Le tableau 1, établi à partir d'analyses du lyophilisat de larves de BOMBYX MORI prélevées après le 8ème jour du 5ème âge, conformément à l'invention, fournit les concentrations d'un certain nombre de composants (quantités relatives).

Le tableau 2 est tire du brevet cité ci-dessus FR1503586, il représente les quantités relatives de certains composants.

Le tableau 3, qui est une analyse comparative des pourcentages de glucides, lipides, protéines, d'une part du produit du brevet cité ci-dessus, d'autre part du produit de l'invention, démontre que ces produits sont totalement différents. En effet, la quantité relative de protéines dans le produit de l'invention est très supérieure, au détriment des lipides.

Ces trois tableaux sont présentés en ANNEXE de la présente description.

On tirera du TABLEAU 1, à titre de proportions caractéristiques de l'extrait, ou substance, relevant de l'invention :
- la proportion protides/lipides de 66,1/14,0 = 4,73, toutjours supérieure à 3,52 dans la période pré-pupale,
- un poids cumulé pour 100 g. de matière sèche (MS) des vitamines B5 et PP, de 15 mg pouvant descendre jusqu'au 10 mg.

On comprendra que seuls les moyens modernes d'analyses ont permis d'optimiser le moment du prélèvement et il est permis d'espérer que des moyens encore plus performants permettront d'affiner encore davantage une ou plusieurs périodes à l'intérieur de celle ci-dessus définie, afin de pouvoir même sélectionner le stade optimal de production d'une vitamine ou d'une hormone particulière ; bien évidemment les conditions d'élevage devront, parallèlement permettre une homogénéité concomittante de la population des chenilles.

A titre de variantes :
- le broyage peut être suivi d'un pressage et/ou d'une filtration,
- on peut ajouter à ce broyat filtré ou non des additifs : conservateurs, antiseptiques, bactéricides, fongicides, antioxydants, huiles essentielles, parfums, ou autres stabilisants,
- à l'un quelconque de ces stades de traitement, le produit peut être congelé sous azote liquide ou non en attendant l'étape suivante,
- le broyat ou le filtrat peut ne pas être congelé ni séché et alors être utilisé pur ou dilué dans un excipient avec ou sans autre principe actif, dès l'instant où il sera stabilisé,
- le séchage peut être, non seulement effectué par lyophilisation, mais aussi par atomisation ou par nébulisation, ou par exemple par séchage sur surface râclée (procédé HATMAKER) ou tout autre moyen au risque de détruire des molécules thermosensibles.

A titre de variante d'extraction, à partir du broyat frais ou du lyophilisat, il est possible d'extraire chimiquement les lipides avec les vitamines liposolubles d'un côté, et les autres composants de l'autre avec, en particulier, les protéines et les vitamines hydrosolubles ; il est aussi possible d'effectuer des extractions aqueuses, des extractions alcooliques.

A titre d'autre variante d'extraction, il est possible d'enlever les glandes séricigènes avant le broyage et on obtiendra, d'une part les glandes séricigènes très riches en acides aminés suivants : tyrosine, glycine, alanine, sérine et que l'on peut broyer très finement jusqu'à obtenir une poudre pulvérulente et, d'autre part, les chenilles amputées de cet organe, avec une teneur en protéines moindre mais sur laquelle on peut procéder identiquement à des extractions diverse : lipidiques, aqueuses, alcooliques entre autres ; l'enlèvement de cette glande séricigène peut avantageusement être réalisée par une centrifugation.

Il est, bien sûr, possible de débactériser la substance obtenue selon le procédé de l'invention, ou ses variantes, de façon plus ou moins poussée selon les domaines d'application.

Des tests de toxicité ont été effectués avec la poudre lyophilisée sur recommandation de l'INSTITUT PASTEUR, par le laboratoire HAZLETON, sur un lot de rats, et se sont avérés négatifs. Aucune toxicité par voie orale n'est apparue, même aux doses les plus élevées.

Des tests d'oxygraphie sont effectués sur l'extrait aqueux : son pouvoir oxygraphique est extrêmement important.

Un essai clinique a été réalisé sur une femme d'une cinquantaine d'années, qui présentait sur l'avant bras gauche, face postérieure, une cicatrice de brûlure, 2ème degré profond, ancienne de 8 mois et, à ses dires, douloureuse au frottement des vêtements, rouge-violacée, boursouflée, dyspigmentée. En quelques jours, l'hyperesthésie avait disparue ; au bout de deux mois, il reste une cicatrice étoilée, complètement indolore, dépigmentée, plane, très souple et ne présentant aucun aspect chéloïdique. Cette femme a, de son propre chef, passé sur sa cicatrice de brûlure, le broyat pur de ver à soie, préparé un an auparavant et conservé au réfrigérateur, sans aucun additif ; l'utilisatrice a seulement noté l'apparition d'un halo plus foncé à la surface du produit et correspondait probablement à l'oxydation des lipides en contact avec l'air. Aucune mauvaise odeur n'est apparue et encore moins l'apparition de moisissures.

Le Demandeur propose pour l'extrait de larves de ver à soie BOMBYX MORI, ci-dessus décrit :
- des applications dermo-cosmétiques : par son haut pouvoir oxygraphique et toutes les propriétés qui en découlent, par son effet tenseur immédiat et prolongé, donc son action anti-rides, par son action sur l'amélioration à effacement complet de certaines tâches dyspigmentées (hypo ou hyper pigmentées), ce qui tendrait à dire que ce serait une action de régulation de la pigmentation cutanée, une amélioration de l'aspect du teint, une action de diminution de la coupe-rose, une action au niveau des vergetures, de même qu'au niveau des petites cicatrices, ainsi qu'une action capillaire certaine de par sa teneur très élevée en acide panthothénique en particulier ; par son effet de reconstitution de la barrière cutanée et donc effet hydratant et amélioration visuelle de la surface cutanée.
- des usages pharmaceutiques : en accélérant les cicatrisations cutanées ou muqueuses, traumatiques ou chirurgicales, récentes ou anciennes, et en améliorant l'élasticité des tissus cicatriciels et leur aspect. On pourrait l'utiliser, en particulier, dans le cas des brûlures profondes ou superficielles (cette invention paraît intéressante pour améliorer les cicatrices chéloïdiques, les hyperesthésies résiduelles) ; par une action au niveau de certains vitiligos en permettant une repigmentation ; par un usage nutritionnel médical à la vue des analyses biochimiques des constituants, très équilibrée en fonction des besoins de l'homme ou de certaines espèces d'animaux (vitamines, oligo-éléments) dont les analyses ont été effectuées par l'INSTITUT PASTEUR de Lyon ; lipides, acides aminés dont les analyses ont été faites par l'INRA de Theix, et par les résultats très encourageants des tests de nutrition à utiliser comme complément alimentaire en particulier dans des situations pathologiques comme les dénutritions post-chirurgicales, ou d'origine cancéreuse, pendant ou après un traitement anti-cancéreux, ou lors de pathologies digestives médicales ou chirurgicales et toutes autres dénutritions relevant de la médecine. A la frontière de la médecine, ce produit pourra être utilisé au cours de régimes demandant une supplémentation en éléments essentiels, par exemple des régimes amaigrissants.

Des usages diététiques "grand public" lors de toutes occasions demandant une supplémentation en éléments essentiels, par exemple, lors de la pratique de sports intensifs, pour ceux qui font de la musculation et qui ont besoin d'un apport accru de protéines ; ou comme aliment de survie par exemple pour les cosmonautes, les spéléologues.

A la limite de la diététique et de la cosmétique, ce produit peut être utilisé comme complément alimentaire de l'application de produits cosmétiques, comme "cosmétique alimentaire".

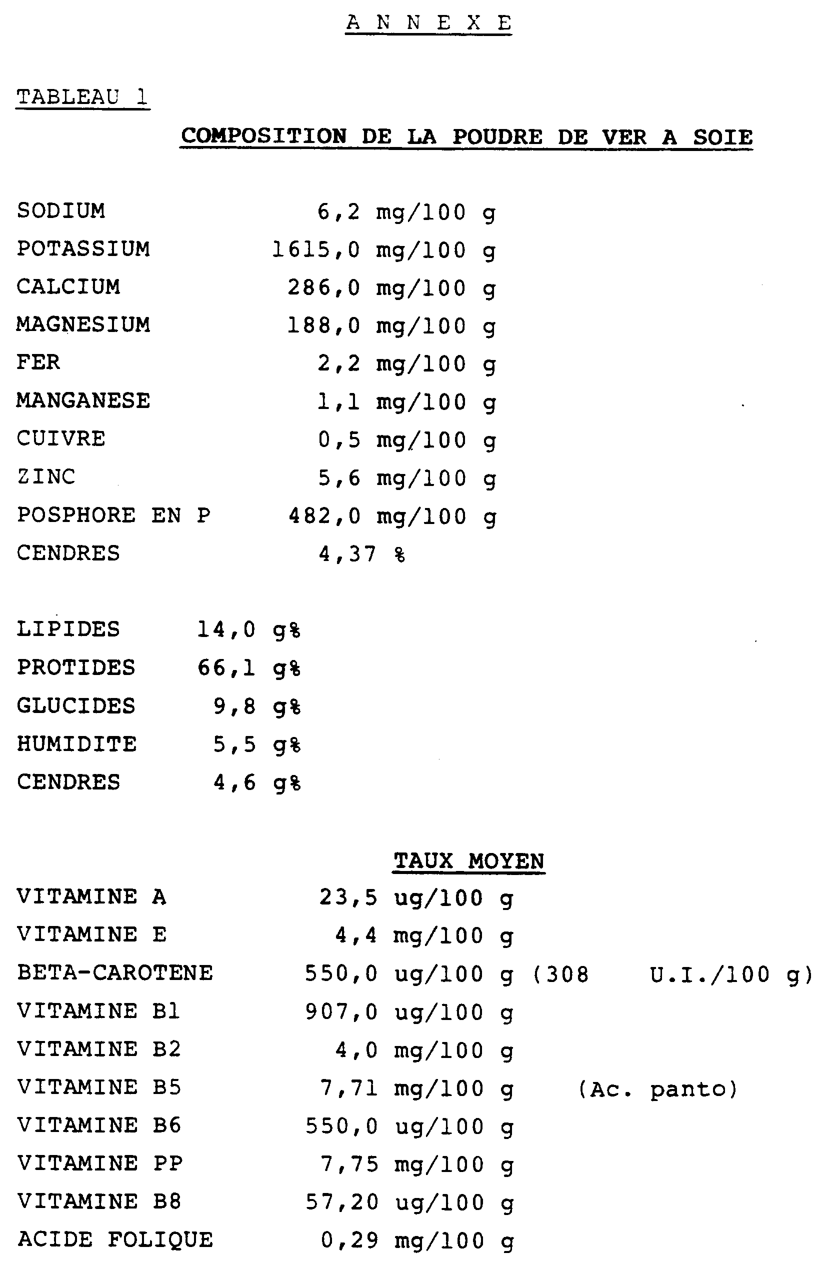

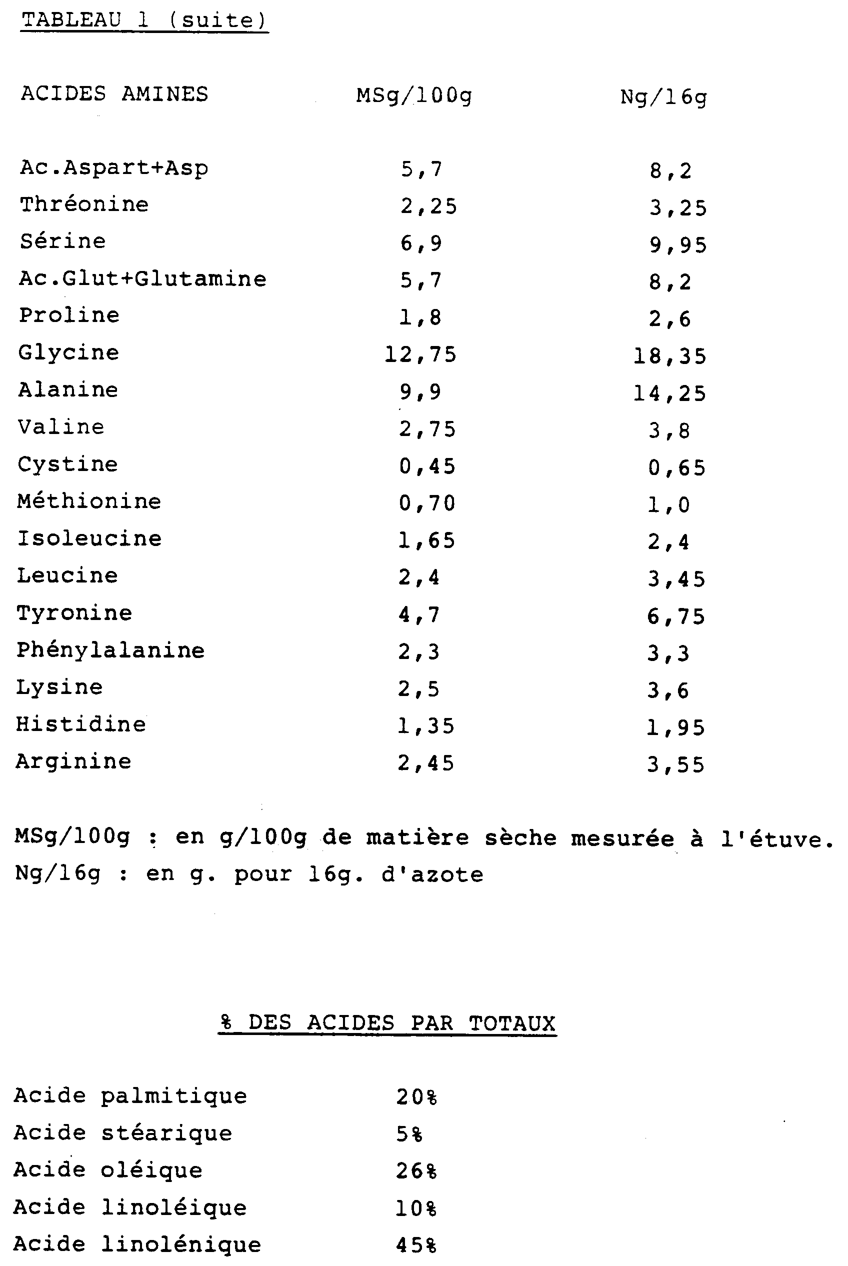

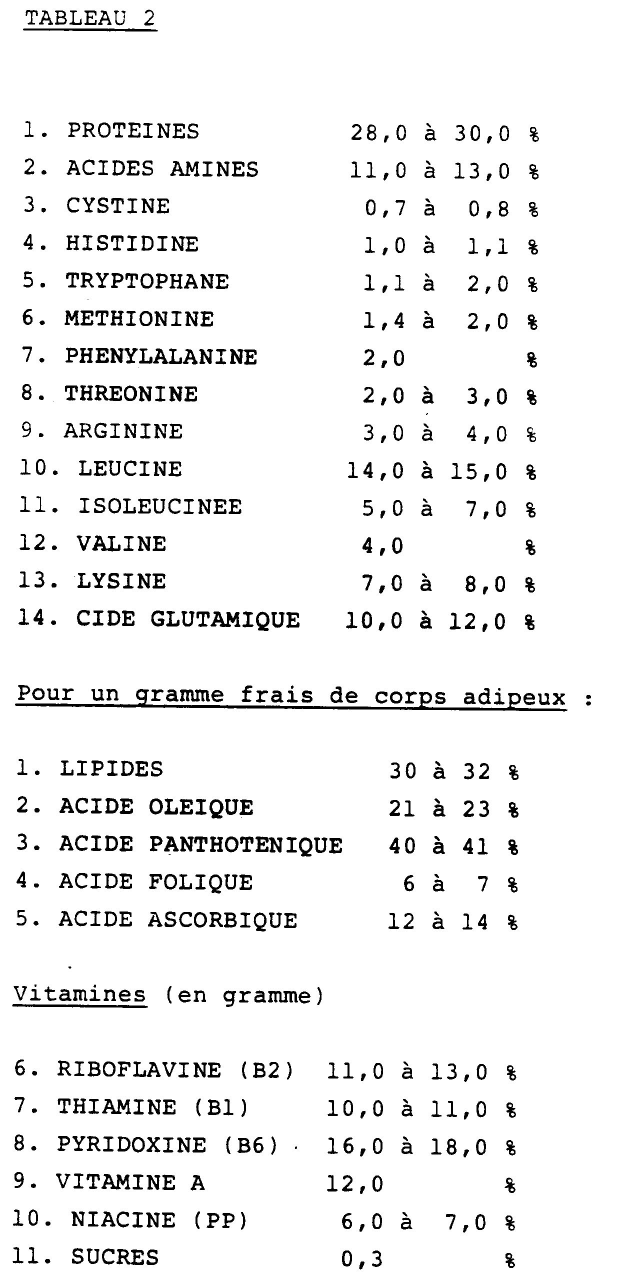

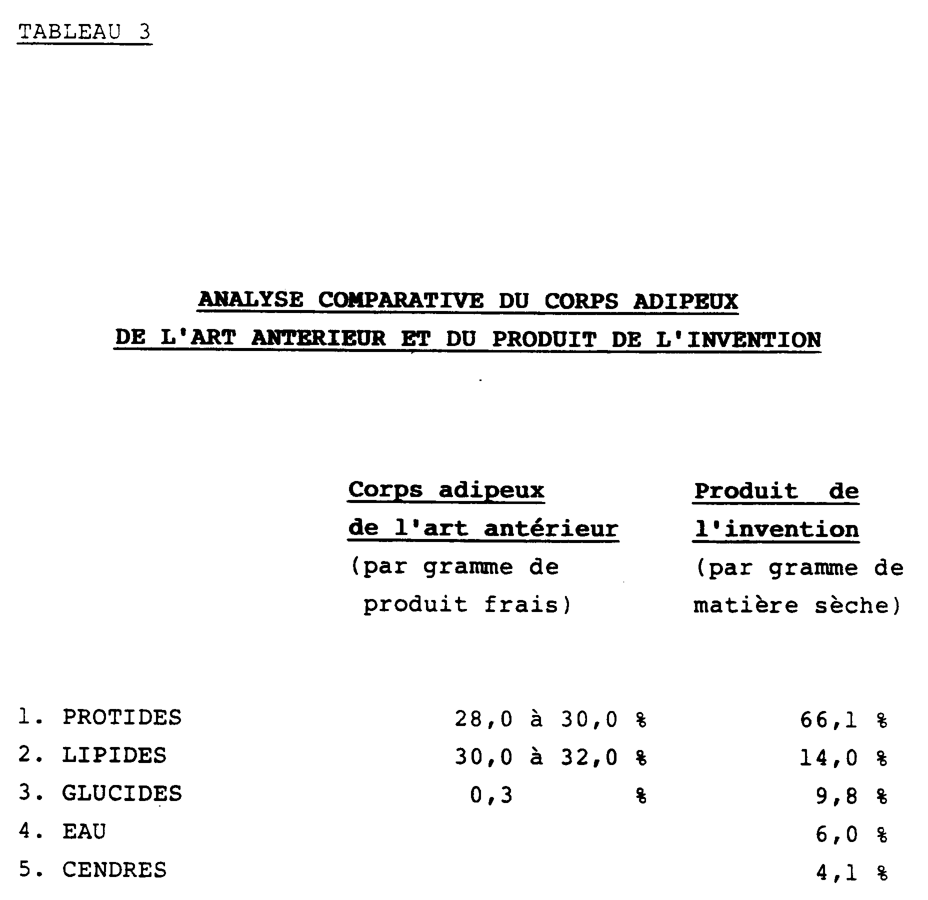

## Revendications

1. Procédé d'obtention d'une substance biologique à partir du ver à soie (Bombyx Mori) élevé en magnanerie et nourri avec des feuilles de mûrier, caractérisé :
en ce qu'il comprend l'étape consistant à sélectionner et récolter celles des chenilles de Bombyx Mori dans leur cinquième age, qui atteignent le stade dit de la "montée" précédant la première métamorphose,

2. Substance résultant du traitement des chenilles récoltées suivant un procédé conforme à la revendication 1, caractérisée :
en ce que, dans la poudre obtenue par un traitement consistant à broyer les chenilles, à sécher le broyât, à le pulvériser et tamiser la poudre obtenue,
- la proportion de protides/lipides est d'environ égale à 4,7, et
- la poids cumulé pour 100g de matière sèche, des vitamines B5 et PP y est d'environ 15mg;

3. Procédé selon la revendication 1, caractérisée :
en ce que l'on procède à l'extraction de la glande séricigène des chenilles sélectionnées et récoltées, pour constituer deux fractions:
- une première fraction, ou extrait, constituée des glandes séricigènes, et
- une seconde fraction constituée des chenilles amputées de leur glande;

4. Procédé selon la revendication 3, caractérisé :
en ce que l'extraction de la glande séricigène est réalisée par centrifugation;

5. Procédé selon la revendication 3, caractérisé :
en ce que l'on soumet l'extrait contenant la glande séricigène à pulvérisation et à tamisage;

6. Utilisation d'une poudre finement pulvérisée et tamisée, dite "soie native", obtenue par le procédé selon la revendication 5, comme produit de maquillage à haut pouvoir couvrant

7. Utilisation d'une poudre grossièrement pulvérisée et tamisée, obtenue par le procédé selon la revendication 5, comme produit de gommage ou de peeling

8. Procédé de traitement d'une poudre conforme à la revendication 2, caractérisé :
en ce qu'une étape ultérieure consiste à soumettre la dite poudre à extraction lipidique au moyen de solvantsen vue d'obtenir une poudre délipidée;

9. Procédé selon la revendication 8, caractérisé :
en ce qu'une étape ultérieure consiste à soumettre la dite poudre délipidée à une extraction acqueuse en vue d'obtenir un résidu délipidé insoluble dans l'eau;

10. Utilisation de la substance délipidée obtenue par le procédé selon la revendication 9, dans la fabrication de médicaments.

## Claims

1. Process for obtaining a biological substance from the silkworm (*Bombyx mori*) raised in a silkworm farm and fed on mulberry leaves, characterized: in that it includes the step consisting of selecting and gathering those caterpillars of *Bombyx mori* in their fifth instar, which reach the stage called "climbing," preceding the first metamorphosis.

2. Substance resulting from the treatment of the caterpillars gathered by a method in accordance with claim 1, characterized:
in that, in the powder obtained by a treatment consisting of crushing the caterpillars, drying the crushed material, pulverizing it and screening the powder obtained.
- the proportion of protides to lipids is approximately equal to 4.7, and
- the accumulated weight per 100 g of dry material of vitamins B5 and PP (niacin) thereof is about 15 mg.

3. Process according to claim 1, characterized:
in that the extraction of the sericigenic gland of the selected and gathered caterpillars is performed, to constitute two fractions:
- a first fraction, or extract, constituted of the sericigenic glands,
and
- a second fraction constituted of the caterpillars deprived of their gland.

4. Process according to claim 3, characterized:
in that the extraction of the sericigenic gland is performed by centrifugation.

5. Process according to claim 3, characterized:
in that the extract containing the sericigenic gland is subjected to pulverization and screening.

6. Use of a finely pulverized and screened powder, called "native silk," obtained by the process according to claim 5, as a make-up powder of high covering power.

7. Use of a roughly pulverized and screened powder obtained by the process according to claim 5, as a gumming or peeling product.

8. Process for treatment of a powder according to claim 2, characterized:
in that a further step consists in subjecting the said powder to an extraction of lipids by means of solvents to obtain a defatted product.

9. Process according to claim 8, characterized:
in that a further step consists in subjecting the said defatted powder to an aqueous extraction for the purpose of obtaining a defatted residue insoluble in water.

10. Use of the defatted substance obtained by the process according to claim 9, in the manufacture of medicaments.

## Patentansprüche

1. Verfahren zum Erhalten einer biologischen Substanz, ausgehend von Seidenraupen (Bombyx Mori), aufgezogen in einer Seidenmupenkammer und ernährt mit Maulbeerblättern,
dadurch gekennzeichnet, daß es den Verfährensschritt umfaßt, jene Raupen von Bombyx Mori auszuwählen, in ihrem fünften Jahr zu sammeln, die das Stadium des "Steigens" erreichen vor der ersten Metamorphose.

2. Substanz, resultierend aus der Behandlung von Seidenraupen, gesammelt entsprechend einem Verfahren gemäß Anspruch 1,
dadurch gekennzeichnet, daß das Pulver, erhalten durch eine Behandlung, bestehend aus einem Zerreiben der Raupen, einem Trocknen des Reibgutes, einem Pulverisieren und Sieben des erhaltenen Pulvers, der Anteil der Protide/Lipide etwa gleich 4,7 ist und das kumulierte Gewicht bei 100 g Trockensubstanz von Vitaminen B5 und PP etwa 15 mg beträgt.

3. Verfahren nach Anspruch 1, dadurch gekennzeichnet, daß die Extraktion der seidenerzeugenden Drüsen von Raupen durchgeführt wird, die ausgewählt und gesammelt wurden, um zwei Fraktionen zu bilden:
eine erste Fraktion oder ein Extrakt, bestehend aus seidenerzeugenden Drüsen, und
eine zweite Fraktion, bestehend aus Raupen ohne ihre Drüse.

4. Verfahren nach Anspruch 3, dadurch gekennzeichnet, daß die Extraktion der seidenerzeugenden Drüse durch Zentrifugieren durchgeführt wird.

5. Verfahren nach Anspruch 3, dadurch gekennzeichnet, daß man den die seidenerzeugende Drüse enthaltenden Extrakt pulverisiert und sortiert.

6. Verwendung eines feinpulverisierten und sortierten Pulvers, der sogenannten nativen Seide, erhalten aus dem Verfahren gemäß Anspruch 5, als Schminksubstanz hoher Deckkraft.

7. Verwendung eines Pulvers, das stark pulverisiert und sortiert ist, erhalten aus dem Verfahren gemäß Anspruch 5, als Gummierungs- oder Peelingmittel.

8. Verfahren zum Behandeln eines Pulvers entsprechend Anspruch 2, dadurch gekennzeichnet, daß ein späterer Verfahrensschritt darin besteht, das genannte Pulver einer Lipidextraktion mittels Lösungsmitteln im Hinblick auf das Erzielen eines endlipidierten Pulvers zu unterwerfen.

9. Verfahren nach Anspruch 8, dadurch gekennzeichnet, daß ein späterer Verfahrensschritt darin besteht, das delipidierte Pulver einer wässrigen Extraktion zu unterwerfen, um einen in Wasser unlöslichen, delipidierten Rückstand zu erhalten.

10. Verwendung der delipidierten Substanz, erhalten aus dem Verfahren gemäß Anspruch 9, bei der Herstellung von Medikamenten.
